# FASCICULE DE BREVET EUROPEEN

(11) **EP 0 688 195 B1**
(45) Date de publication et mention de la délivrance du brevet: **04.04.2001**
(21) Numéro de dépôt: 95907024.4
(22) Date de dépôt: 09.01.1995
(51) Int. Cl.: A61F 2/01

(54) **FILTRE ENDOVASCULAIRE AVEC PATTES DE FIXATION APLATIES**
ENDOVASKULÄRER FILTER MIT ABGEFLACHTEN FIXIERUNGSFINGERN
ENDOVASCULAR FILTER WITH FLAT FIXING BRANCHES

(30) Priorité: 10.01.1994 FR 9400339
(43) Date de publication de la demande: 27.12.1995
(73) Titulaire: BENTEX TRADING S.A., L-2311 Luxembourg (LU)
(72) Inventeur: LEFEBVRE, Jean-Marie, F-59800 Lille (FR)
(74) Mandataire: 't Jong, Bastiaan Jacobus
(86) Numéro de dépôt international: FR9500027
(87) Numéro de publication internationale: WO9518582

(56) Documents cités:
- EP-A- 0 350 043
- EP-A- 0 448 891
- EP-A- 0 462 008
- FR-A- 2 672 487
- US-A- 3 334 629
- US-A- 5 059 205

## Description

La présente invention concerne tous les dispositifs qui sont destinés à être placés de manière provisoire ou définitive dans un vaisseau et qui comportent des pattes aplaties coupant le flux sanguin ; elle concerne notamment les filtres empêchant la migration des caillots sanguins. Plus précisément la présente invention concerne l'assemblage entre-elles des pattes aplaties , dénommées pattes de fixation dans le présent texte,dont sont pourvus lesdits dispositifs et qui remplissent soit des fonctions de filtration soit des fonctions de fixation sur la paroi du vaisseau soit le cumul de ces deux fonctions.

On connaît par la demande de brevet européen EP.462.008 un dispositif filtrant destiné à être placé dans un vaisseau sanguin, et comportant des pattes de fixation aplaties, qui sont obtenues par laminage d'un fil réalisé en alliage de qualité médicale. Ces pattes de fixation sont rendues solidaires entre-elles par soudure de leurs extrémités sur une pièce de liaison. Lorsque ce dispositif filtrant est introduit dans un vaisseau , les extrémités des pattes de fixation aplaties subissent deux types de contraintes : d'une part elles subissent une contrainte mécanique, qui tend à les désolidariser de la pièce de liaison, et d'autre part, elles subissent, au contact du flux sanguin, une corrosion chimique , qui vient détériorer dans le temps les points de soudure permettant la fixation de ces extrémités sur la pièce de liaison.

Ces deux contraintes ont pour résultat de fragiliser dans le temps la fixation, au niveau de chaque pièce de liaison, des extrémités des pattes aplaties du filtre. Il y a donc un risque grandissant que les extrémités des pattes du filtre se désolidarisent entre-elles, le filtre devenant alors inefficace et risquant d'endommager de manière irréversible la paroi veineuse. La fragilité d'un tel dispositif filtrant au niveau des extrémités de ces pattes de fixation nuit à la longévité d'un tel dispositif , une fois implanté dans le vaisseau sanguin.

Le but de la présente invention est donc de proposer un dispositif du type de celui de la demande de brevet européen EP.462.008, mais qui en pallie les inconvénients.

Ce but est parfaitement atteint par le dispositif de l'invention, qui conformément au dispositif de la demande de brevet EP.462.008 est destiné à être placé dans un vaisseau, et est muni de pattes de fixation, qui sont formées chacune par une pièce large et plate, et qui coupent le flux sanguin lorsque le dispositif est placé dans le vaisseau.

De manière caractéristique selon l'invention , les extrémités des pattes de fixation sont bloquées à l'intérieur d'au moins une tête ogivale , au moyen d'une pièce de blocage , et la forme de ces extrémités est adaptés aux périphéries interne et externe respectivement de la tête ogivale et de la pièce de blocage.

La première fonction de la tête ogivale et de la pièce de blocage est de réaliser une liaison mécanique des extrémités des pattes de fixation, qui soit fiable et empêche tout risque de désolidarisation de ces extrémités, sous l'effet des contraintes mécaniques exercées sur les pattes de fixation du dispositif. Etant donné par ailleurs que dans la version la plus générale du dispositif de l'invention, on ne soude pas les extrémités des pattes de fixation du dispositif, on a par la même occasion résolu le problème de corrosion chimique de ces points de soudure, au contact du flux sanguin.

Pour réaliser l'assemblage entre-elles des extrémités des pattes de fixation du dispositif de l'invention au moyen de la tête ogivale et de la pièce de blocage, on commence par positionner les extrémités des pattes de fixation à l'intérieur de la tête ogivale. Ensuite, on vient insérer à force la pièce de blocage dans la tête ogivale, réalisant par là même le blocage des extrémités des pattes de fixation. En théorie, l'effet de blocage étant suffisamment fiable, il n'est pas nécessaire de souder les extrémités des pattes de fixation. En pratique , lorsque l'on réalise cette opération d'assemblage, I 'introduction de la pièce de blocage provoque un déplacement relatif des extrémités des pattes de fixation, qui viennent très légèrement se chevaucher à l'intérieur de la tête ogivale. Il en résulte un défaut de parallélisme des pattes de fixation entre-elles qui est préjudiciable à la mise en oeuvre du filtre. En effet, lorsque les pattes de fixation d'un tel dispositif sont à l'état replié, il est important que celles-ci puissent être dans un position parfaitement parallèle entre-elles, en sorte de permettre l'introduction du dispositif, au moyen d'un cathéter, dans le vaisseau sanguin.

Pour pallier cet inconvénient, il est donc préférable de souder les extrémités des pattes de fixation , soit directement sur la face interne de la tête ogivale ou sur la face externe de la pièce de blocage , soit encore de souder les extrémités des pattes de fixation entre-elles, le long de leurs bords longitudinaux. Cette soudure des extrémités des pattes de fixation a pour principale fonction d'éviter un déplacement relatif des pattes de fixation lors de l'introduction de la pièce de blocage dans la tête ogivale. Accessoirement , la soudure des extrémités permet d'augmenter la fiabilité de la solidité mécanique du filtre. Il est important de noter , que dans ce cas , la tête ogivale et la pièce de blocage remplissent une deuxième fonction qui est de protéger du flux sanguin les points de soudure des extrémités de fixation , et par là même de diminuer les risques de détérioration par corrosion chimique.

Selon une première variante particulière de réalisation, la pièce de blocage et la tête ogivale sont des pièces cylindriques ; chaque patte de fixation possède une extrémité légèrement courbée en sorte de s'adapter à la périphérie interne de la tête ogivale et à la périphérie externe de la pièce de blocage.

Selon une seconde variante de réalisation, les périphéries interne et externe respectivement de la tête ogivale et de la pièce de blocage forment des polygones réguliers, dont le nombre de côtés est égal au nombre de pattes de fixation du filtre , et dont les côtés sont parallèles deux à deux.

La présente invention sera mieux comprise à la lecture de la description qui va être faite d'un exemple de réalisation d'un filtre à usage médical comportant des pattes de fixation aplaties,et illustré par le dessin annexé dans lequel :
La figure 1 est une vue schématique de côté du filtre à l'intérieur de la veine,
La figure 2 est une vue en coupe du filtre de la figure 1,
Les figures 3A et 3B sont des vues en coupe transversales d'une patte de fixation obtenue respectivement par écrasement d'un fil rond (figure 3A) et par découpe dans une plaque métallique (figure 3B),
La figure 4 est une représentation partielle en perspective d'une patte de fixation avec pli anguleux ,
La figure 5A est une vue en coupe transversale de la tête d'un filtre, dans lequel les pattes de fixation ont été courbées dans leur largeur,
et la figure 5B est une vue en coupe transversale de la tête d'un filtre, dans lequel les périphéries interne et externe respectivement de la tête ogivale et de la pièce de blocage sont hexagonales.

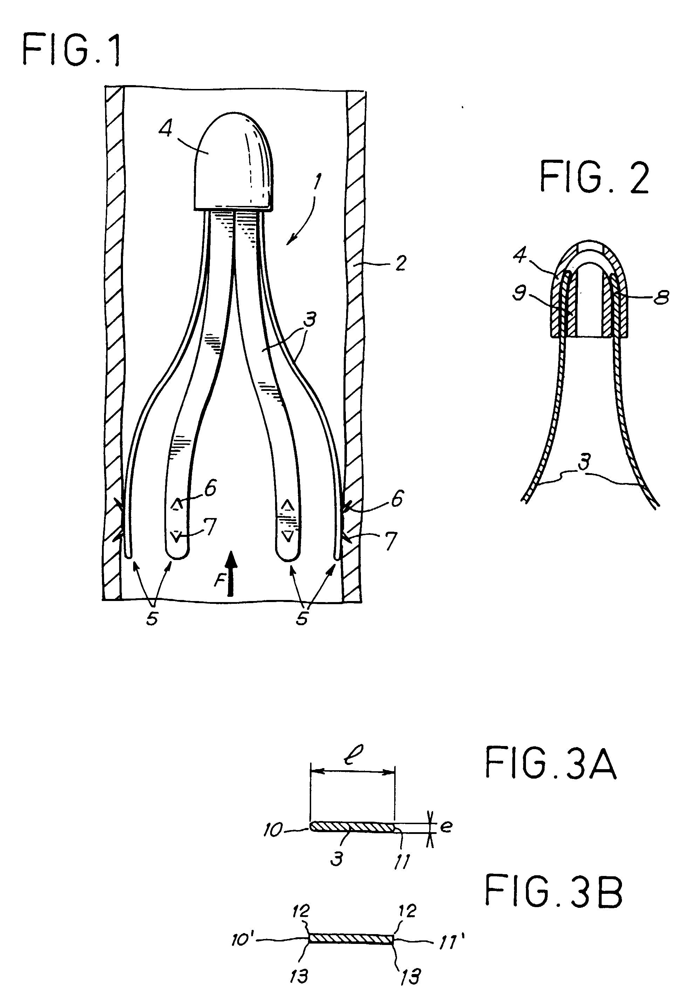

On a représenté sur la figure 1 le filtre 1 lorsqu'il est placé à l'intérieur d'une veine 2 qui est parcourue par le flux sanguin dans le sens de la flèche F. Ce filtre 1 a une forme générale conique avec les pattes de fixation 3 qui sont fixées à une tête ogivale 4. Les extrémités libres 5 de chaque patte de fixation 3 ont un bout arrondi et comportent deux dents 6,7 dirigées , selon la direction générale de la patte, de manière antagoniste.

Chaque patte 3 est constituée d'une lame plate dont l'extrémité distale 8 est bloquée en position à l'intérieur de la tête ogivale 4 par une pièce cylindre de blocage 9 (figure 2).

Dans un exemple préféré de réalisation, le filtre 1 comporte six pattes latérales 3 qui sont disposées symétriquement et accolées les unes aux autres sur la périphérie intérieure de la tête ogivale 4 et bloquées en position par introduction de la pièce de blocage 9.

Pour la réalisation d'une patte 3, on part d'un fil métallique de section circulaire auquel on fait subir une opération d'aplatissement par écrasement à chaud.

On comprend que , dans ces conditions , les bords longitudinaux 10,11 de la patte 3 ont une forme arrondie (figure 3A).

On a représenté sur les figures 3A et 3B les sections transversales respectives de deux pattes de fixation 3 : la première (figure 3A) est obtenue par écrasement d'un fil métallique conformément à la demande de brevet EP.462.008, tandis que la seconde (figure 3B) est obtenue par simple découpe d'une plaque métallique, conformément notamment à l'enseignement du document FR 2 570 288. Dans cette dernière, on constate que les bords longitudinaux 10', 11' comportent des arêtes vives 12, 13.

Lors du passage du flux sanguin à l'intérieur du vaisseau 2, les pattes de fixation 3 font office de filtre pour caillots sanguins. Cependant dans le cas où ces pattes 3 présentent des arêtes vives 12, 13 comme c'est le cas du filtre décrit dans le document FR 2 570 288, on constate que dans certaines conditions il se forme des caillots sanguins du fait même de la présence de ces arêtes 12, 13.

Ce phénomène n'est absolument pas observé lorsque les bords latéraux 10, 11 des pattes de fixation 3 ont une forme arrondie, comme cela est représenté à la figure 3A.

Le principe des crochets 6, 7, situés aux extrémités libres 5 des pattes 3 est connu par le document FR 2 660 189.

Avantageusement chaque crochet 6, 7 est obtenu par découpe et pliage d'une forme en V réalisée dans la zone centrale de l'extrémité libre 5 de chaque patte 3. Le fait que l'on procède, pour la formation des crochets, à une découpe ne présente pas d'inconvénient dans la mesure où les crochets viennent se fixer dans le vaisseau 2 et ne sont donc pas placés d'une manière permanente dans le flux sanguin.

Dans un exemple précis de réalisation, chaque patte de fixation 3 a une largeur l qui est de l'ordre de 2 mm et une épaisseur e de l'ordre de 0,2 mm. Elle est obtenue par aplatissement d'un fil métallique présentant en section transversale un diamètre de l'ordre de 0,7 mm.

Après aplatissement, les lames métalliques obtenues sont préformées et traitées thermiquement pour avoir en position normale la forme courbe en S telle qu'elle apparaît à la figure 1.

On comprend que lors de l'opération d'aplatissement du fil métallique, la lame aplatie obtenue, constitutive d'une patte de fixation , possède un certain sens, au même titre qu'une plaque métallique sortant de laminage. Il importe donc que, lors des opérations de préformage, destinées à donner à la lame aplatie la forme requise pour son utilisation comme patte de fixation dans le dispositif à usage médical de l'invention, la lame aplatie possède toujours la même orientation. Ainsi on évite les risques d'asymétrie.

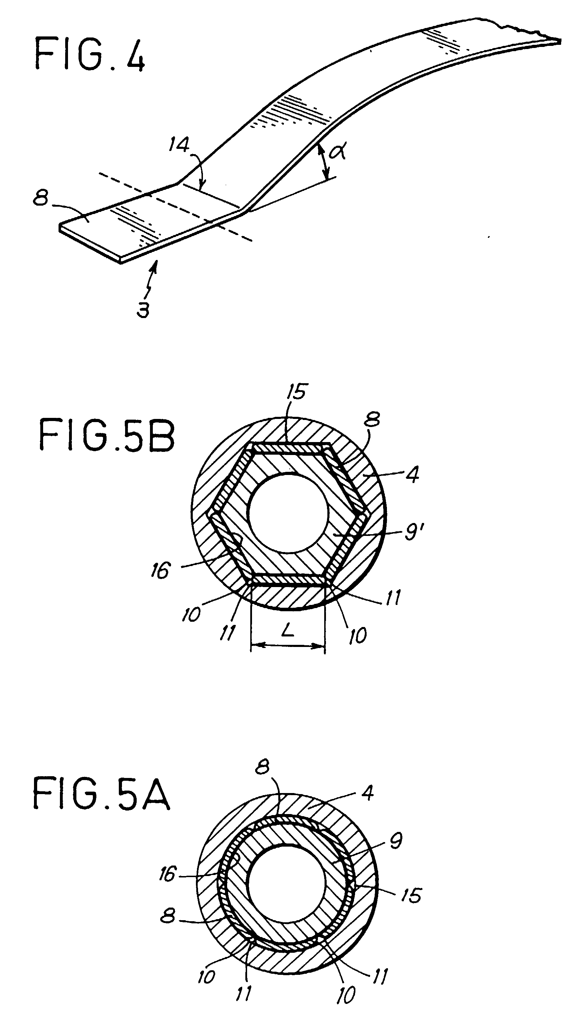

Pour mettre en place le filtre de la figure 1 à l'intérieur d'une veine, on le fait cheminer dans un cathéter. La présence de sang à l'intérieur du cathéter induit nécessairement un réseau de fibrines, qui enveloppe le filtre et gêne l'expansion des pattes de fixation 3. Or il est nécessaire que le filtre puisse s'ouvrir pour que les pattes de fixation puissent assurer leur fonction de filtrage et également que la force de pression exercée sur les parois de la veine, par les pattes de fixation 3, soit suffisante pour permettre un ancrage du filtre dans cette paroi, par l'intermédiaire des crochets 6,7. Si l'on veut augmenter cette force de pression, il est nécessaire, compte tenu de la courbe en S des pattes 3 du filtre 1, d'utiliser des lames dont l'épaisseur est plus importante, ce qui pose un problème d'encombrement lorsque le filtre est replié. Or il est impératif que cet encombrement soit minimum. Par conséquent, dans le but d'obtenir une meilleure ouverture du filtre lors de son éjection du cathéter et d'augmenter la force de pression exercée par les pattes de fixation 3 sur la paroi de la veine, sans augmenter l'épaisseur des lames métalliques utilisées, on fait subir à chaque patte de fixation 3, après aplatissement, un traitement mécanique de pliage de manière à réaliser un pli anguleux 14, d'angle a, dans une zone proximale de son extrémité distale 8, tel que cela est illustré sur la figure 4. Dans un exemple précis de réalisation, la mesure de l'angle α valait environ 30°. Ce pli anguleux 14 confère à la patte de fixation 3 un effet de ressort supplémentaire. Sur la figure 4, on a schématisé par une ligne en pointillés, la limite de la tête ogivale 4, lorsque l'extrémité distale 8 est bloquée en position à l'intérieur de celle-ci.

Dans un premier mode particulier de réalisation, avant son introduction à l'intérieur de la tête ogivale 4, chaque patte de fixation 3 subit, au moins vers son extrémité distale 8, un traitement mécanique de pressage destiné à lui donner une légère courbure sur sa largeur. Cette disposition particulière permet de positionner les unes à côté des autres les extrémités distales 8 de toutes les pattes de fixation 3, entrant dans la composition du filtre 1, selon la périphérie interne 15 de la tête ogivale 4, et selon la périphérie externe 16 de la pièce cylindre de blocage 9 (figure 5A).

Dans un deuxième mode particulier de réalisation illustré à la figure 5B, les périphéries interne 15 et externe 16 respectivement de la tête ogivale 4 et de la pièce de blocage 9', sont des polygones réguliers, en l'occurrence des hexagones, dont le nombre de côtés équivaut au nombre de pattes de fixation 3 du filtre. Plus particulièrement, la largeur L d'un côté de la périphérie externe 16 de la pièce de blocage 9' est égale à la largeur de chaque patte de fixation 3 ; la tête ogivale 4 et la pièce de blocage 9' sont positionnées l'une par rapport à l'autre de telle sorte que les côtés de leur périphérie respectivement interne 15 et externe 16 soient parallèles deux à deux.

De préférence, dans les deux modes particuliers de réalisation des figures 5A et 5B, lorsque les extrémités distales 8 des pattes de fixation 3 sont positionnées à l'intérieur de la tête ogivale 4, on réalise, préalablement à la mise en place de la pièce de blocage 9,9' , une opération de soudage au laser des extrémités distales 8 entre elles, par l'intermédiaire de leurs bords longitudinaux 10 et 11. Ainsi lors de la mise en place de la pièce de blocage 9,9' , on évite tout risque de déplacement de pattes de fixation 3 par rapport à la périphérie externe de la pièce de blocage 9, 9'. Les pattes de fixation n'ont donc pas tendance à se chevaucher lors de l'assemblage et le filtre 1 reste ainsi parfaitement symétrique.

La présente invention n'est pas limitée aux modes de réalisation qui viennent d'être décrits à titre non exhaustif. En particulier le dispositif à usage médical peut comporter un nombre différent de pattes de fixation. Il est en outre envisageable dans le cadre de l'invention de réaliser un dispositif filtrant à centrage automatique similaire à celui de la demande de brevet européen EP.462.008. Dans ce cas , on utilise deux têtes ogivales , et deux pièces de blocage pour assembler les deux extrémités de chaque patte de fixation du filtre avec les extrémités correspondantes des autres pattes de fixation. Enfin la soudure des pattes de fixation peut être réalisée sur la face interne ou externe respectivement de la tête ogivale ou de la pièce de blocage.

## Revendications

1. Dispositif à usage médical, destiné à être placé dans un vaisseau, muni de pattes de fixation (3), qui sont formées chacune par une pièce large et plate et qui coupent le flux sanguin lorsque le dispositif est placé dans le vaisseau , caractérisé en ce que les extrémités (8) des pattes de fixation (3) sont bloquées à l'intérieur d'au moins une tête ogivale (4) au moyen d'une pièce de blocage (9,9'), et en ce que la forme de ces extrémités (8) est adaptée aux périphéries interne (15) et externe (16) respectivement de la tête ogivale (4) et de la pièce de blocage (9,9').

2. Dispositif selon la revendication 1 caractérisé en ce que la pièce de blocage (9) et la tête ogivale (4) étant des pièces cylindriques , chaque patte de fixation (3) possède une extrémité (8) légèrement courbée en sorte de s'adapter à la périphérie interne (15) de la tête ogivale et à la périphérie externe (16) de la pièce de blocage (9).

3. Dispositif selon la revendication 1 caractérisé en ce que les périphéries interne (15) et externe (16) respectivement de la tête ogivale (4) et de la pièce de blocage (9') forment des polygones réguliers , dont le nombre de côtés est égal au nombre de pattes de fixation (3) du filtre , et dont les côtés sont parallèles deux à deux.

4. Dispositif selon la revendication 3 caractérisé en ce que la largeur L d'un côté de la périphérie externe (16) de la pièce de blocage (9') est égale à la largeur 1 d'une patte de fixation (3).

5. Dispositif selon l'une des revendications 1 à 4 caractérisé en ce que les extrémités (8) des pattes de fixation (3) sont soudées entre-elles, par l'intermédiaire de leurs bords longitudinaux (10,11) et / ou sur la face interne de la tête ogivale (4) ou sur la face externe de la pièce de blocage (9).

6. Dispositif selon l'une des revendications 1 à 5 caractérisé en ce que chaque patte de fixation (3) comporte un pli anguleux (14) d'angle α dans une zone proximale de son extrémité (8).

7. Dispositif selon l'une des revendications là 6 , caractérisé en ce que chaque patte de fixation (3) est formée d'une pièce longue et plate dont les bords longitudinaux (10,11) sont arrondis.

8. Dispositif selon la revendication 7 caractérisé en ce que chaque patte de fixation (3) est formée d'une pièce longue et plate obtenue par écrasement d'un fil métallique de section circulaire.

## Claims

1. Device for medical use, intended to be placed in a vessel, provided wich fixing legs (3), which are each formed of a broad and flat piece and which interrupt the blood flow when the device is placed in the vessel, characterised by the fact that the ends (8) of the fixing legs (3) are locked inside at least one ogival head (4) by means of a locking piece (9, 9'), and by the fact that the shape of these ends (8) fits the internal (15) and external (16) peripheries of the ogival head (4) and of the locking piece (9, 9') respectively.

2. Device as described in claim 1, characterised by the fact that the locking piece (9) and the ogival head (4) being cylindrical pieces, each fixing leg (3) has a slightly curved end (8) so as to fit the internal periphery (15) of the ogival head and the external periphery (16) of the locking piece (9).

3. Device as described in claim 1, characterised by the fact that the internal (15) and external (16) peripheries of the ogival head (4) and of the locking piece (9') respectively form regular polygons, the number of sides of which is equal co the number of fixing legs (3) of the filter and the sides of which are parallel in pairs.

4. Device as described in claim 3, characterised by the fact that the width L of a side of the excernal periphery (16) of the locking piece (9') is equal to the width 1 of a fixing leg (3).

5. Device as described in one of claims 1 to 4, characterised by the fact that the ends (8) of the fixing legs (3) are welded together, by means of their longitudinal edges (10, 11) and/or on the internal face of the ogival head (4) or on the external face of the locking piece (9).

6. Device as described in one of claims 1 to 5, characterised by the fact that each fixing leg (3) includes an angular fold (14) of angle α in a zone close to its end (8).

7. Device as described in one of claims 1 to 6, characterised by the fact that each fixing leg (3) is formed of a long and flat piece of which the longitudinal edges (10, 11) are rounded.

8. Device as described in claim 7, characterised by the fact that each fixing leg (3) is formed of a long and flat piece obtained by crushing a metal wire of circular section.

## Patentansprüche

1. Für das Anbringen in einem Gefäß bestimmte Vorrichtung zur medizinischen Anwendung, mit Fixierungsfingern (3), die jeweils von einem breiten und flachen Teil gebildet sind und den Blutstrom schneiden, wenn die Vorrichtung in dem Gefäß angebracht ist,
**dadurch gekennzeichnet,**
daß die Enden (8) der Fixierungsfinger (3) im Innern wenigstens eines spitzbogenformigen Kopfes (4) mit Hilfe eines Klemmstücks (9, 9') arretiert sind,
und daß die Form dieser Enden (8) an die innere Peripherie (15) und die äußere Peripherie (16) des spitzbogenförmigen Kopfes (4) bzw. des Klemmstücks (9, 9') angepaßt ist.

2. Vorrichtung nach Anspruch 1, dadurch gekennzeichnet, daß das Klemmstück (9) und der spitzbogenförmige Kopf (4) zylindrische Teile sind und jeder Fixierungsfinger (3) ein leicht gekrümmtes Ende (8) besitzt, so daß es sich an die innere Peripherie (15) des spitzbogenförmigen Kopfes und an die äußere Peripherie des Klemmstücks (9) anpaßt.

3. Vorrichtung nach Anspruch 1, dadurch gekennzeichnet, daß die innere Peripherie (15) und die äußere Peripherie (16) des spitzbogenförmigen Kopfes (4) bzw. des Klemmstücks (9') regelmäßige Polygone bilden, deren Seitenzahl gleich der Zahl der Fixierungsfinger (3) des Filters ist und deren Seiten parallel zueinander verlaufen.

4. Vorrichtung nach Anspruch 3, dadurch gekennzeichnet, daß die Breite L einer Seite der inneren Peripherie (15) des Klemmstücks (9') gleich der Breite 1 eines Fixierungsfingers (3) ist.

5. Vorrichtung nach einem der Ansprüche 1 bis 4, dadurch gekennzeichnet, daß die Enden (8) an ihren Längsrändern (10, 11) und/oder auf der Innenseite des spitzbogenformigen Kopfes (4) oder der Außenseite des Klemmstücks (9) miteinander verschweißt sind.

6. Vorrichtung nach einem der Ansprüche 1 bis 5, dadurch gekennzeichnet, daß jeder Fixierungsfinger (3) in einer proximalen Zone seines Endes (8) einen kantigen Falz (14) aufweist, der den Winkel u hat.

7. Vorrichtung nach einem der Ansprüche 1 bis 6, dadurch gekennzeichnet, daß jeder Fixierungsfinger (3) aus einem langen und flachen Teil gebildet ist, dessen Längsränder (10, 11) abgerundet sind.

8. Vorrichtung nach Anspruch 7, dadurch gekennzeichnet, daß jeder Fixierungsfinger (3) aus einem langen und flachen Teil gebildet ist, das durch Quetschen eines Metalldrahts mit kreisrundem Querschnitt gewonnen wird
